**Europäisches Patentamt**

**European Patent Office**

**Office européen des brevets**

⑱

(11) Publication number: **0 051 063**
**B1**

(12) # EUROPEAN PATENT SPECIFICATION

(45) Date of publication of patent specification: **07.11.84**

(21) Application number: **81900412.8**

(22) Date of filing: **03.02.81**

(86) International application number:
**PCT/SE81/00033**

(87) International publication number:
**WO 81/02308 20.08.81 Gazette 81/20**

(51) Int. Cl.³: **C 12 P 5/02,** C 02 F 3/28,
C 12 M 1/02

(54) A PROCESS AND AN APPARATUS FOR CONVERTING ORGANIC MATERIAL ALONG A MICRO-BIOLOGICAL ROUTE.

(30) Priority: **05.02.80 SE 8000923**

(43) Date of publication of application:
**12.05.82 Bulletin 82/19**

(45) Publication of the grant of the patent:
**07.11.84 Bulletin 84/45**

(84) Designated Contracting States:
**AT CH DE FR GB LI NL SE**

(56) References cited:
**CH-A- 612 655**
**DE-A-2 249 137**
**DE-A-2 331 192**
**DE-A-2 531 598**
**DE-B-2 332 218**
**DE-B-2 924 465**
**SE-B- 397 955**

(73) Proprietor: **AB SORIGONA**
**Box 134**
**S-245 00 Staffanstorp (SE)**

(72) Inventor: **HUSS, Lennart**
**Jupitervägen 1**
**S-245 00 Staffanstorp (SE)**

(74) Representative: **Burman, Tore et al**
**Bergling & Sundbergh AB P.O. Box 7645**
**S-103 94 Stockholm (SE)**

Courier Press, Leamington Spa, England.

## Description

Technical Field

The present invention relates to a process of converting in a reaction vessel containing a movable bed organic materials in a microbiological way under the formation of gas.

When using microorganisms for the manufacture of different types of products it is essential that the contents of active microorganisms in the reaction vessel is kept as high as possible.

Background Art

In accordance with conventional prior art the contents of organisms is controlled by means of static sedimentation, floatation, centrifugation or filtration, the microorganisms being separated from the discharged liquid phase and returned to the reaction zone as biosludge. Limiting factors in this known technique is for example the sedimentation properties, the centrifugation or filtration performance of the biosludge formed. Static sedimentation, which is the technique most frequently used involves the limitation that the biosludge cannot take up more than about 50% of the volume in a sedimented state in order that static sedimentation shall operate in a satisfactory manner. This volume corresponds to a concentration of bio mass of 5—10 kgs DS (dry substance) per m³ of reaction vessel.

In another type of known technique there is used a reaction vessel containing a packing, the bio mass being fixed to a solid surface structure, for example a bed of stone materials or plastics. A limiting factor in this case is the fact that no more than about 100—200 m² surface per m³ of reaction vessel can be obtained if blocking of the cavities is to be prevented. Also this specific surface corresponds to a concentration of bio mass of 5—10 kgs DS/m³ of reaction vessel. For such technique see f.i. Swedish patent specification Ser. No. 7605005-3.

In a third variant of the known technique there is used a suspensible bed in a reaction vessel, the bed being fluidized by means of an upward flow of gases or liquid. (See f.i. Swiss patent specification Ser. No. 612.655.) However, this system provides for a pronounced practical limitation, in that the distribution of the fluidization flow must be exactly uniform across all of the lower part of the reaction zone cross section, which is very difficult to obtain in a large reaction vessel. Moreover, such fluid bed system calls for careful selection of bed material with regard to grain size and distribution, as is well known in the art.

Summary of the Invention

Thus, the object of the present invention is to provide a process and an apparatus whereby organic materials in a microbiological way can be converted under the generation of gas while avoiding the disadvantages associated with for for example the above-described conventional techniques.

To this end there is provided by the invention a process wherein the conversion is performed in a reaction vessel by means of microorganisms which are grown on a movable bed of inorganic granular material and which, furthermore, are present in a free state in the form of micro flocks, the conversion taking place in a continuous aqueous phase and substrate for the microorganisms being supplied to the bed in connection to a mechanical stirrer. The gases generated from the conversion are utilized for expanding the bed, and the free microflocks are externally separated at a place outside the reaction vessel from liquid discharged therefrom and are then returned to the reaction zone.

The stirrer is suitably arranged so as to impart to the bed material a downward movement, preferably all the way down to the bottom of the reaction vessel, and it is preferred to introduce the substrate into the vessel within the part of the bed subject to such downward movement. This results in efficient fluidization of the bed due to the generation of gases therein. Thus, by the combined pneumatic and hydraulic mixing effects an optimized overall process results.

In a preferred embodiment of the process of the invention there is used a bed based on an organic granular material having a grain size lying within the range about 0.05—5 mm. This material constituting the bed can be sand, calcium, carbonate, calcium hydroxide or dolomite or the like.

The invention also relates to an apparatus wherein this process can be performed, and this apparatus includes a reaction vessel, a stirrer arranged therein, means for supplying liquid containing substrate and means for discharging liquid and for recirculation of free active material. To obtain the object of the invention the apparatus includes a movable bed of inorganic granular material arranged in the reaction vessel, said bed being intended for supporting growing microorganisms for conversion of the organic material.

The movable bed is preferably made of materials described above in connection with the process.

The stirrer used is preferably constituted by a radial or axial turbine or propeller placed at the lower end of the reaction vessel and suitably positioned centrally therein.

At rest the movable bed suitably occupies 20—80% of the volume of the reaction vessel, preferably about 40—60%.

The reaction vessel of the apparatus of the invention is suitably provided with interior vertical baffles over 20—100% of the height of the vessel, and these baffles can be present in a number of from 1—10.

The invention is generally applicable on a number of different types of microbiological processes depending on type of substrate and type of microorganism. As examples there may be mentioned microorganisms of a monoculture, such as *Saccaromyces Services*, to form ethanol from carbohydrate substrates, or of a multiculture, such as in the conversion of complex organic substrates to methane gas and carbon dioxide. Another application of the invention is in denitrification of nitrate-rich aqueous substrates.

### Example

The invention will now be further described by a non-limiting example in connection with the appended drawing, wherein

Fig. 1 shows diagramatically a vertical cross section through an embodiment of the apparatus according to the invention, and

Fig. 2 shows diagrammatically the circulatory features in the embodiment according to Fig. 1.

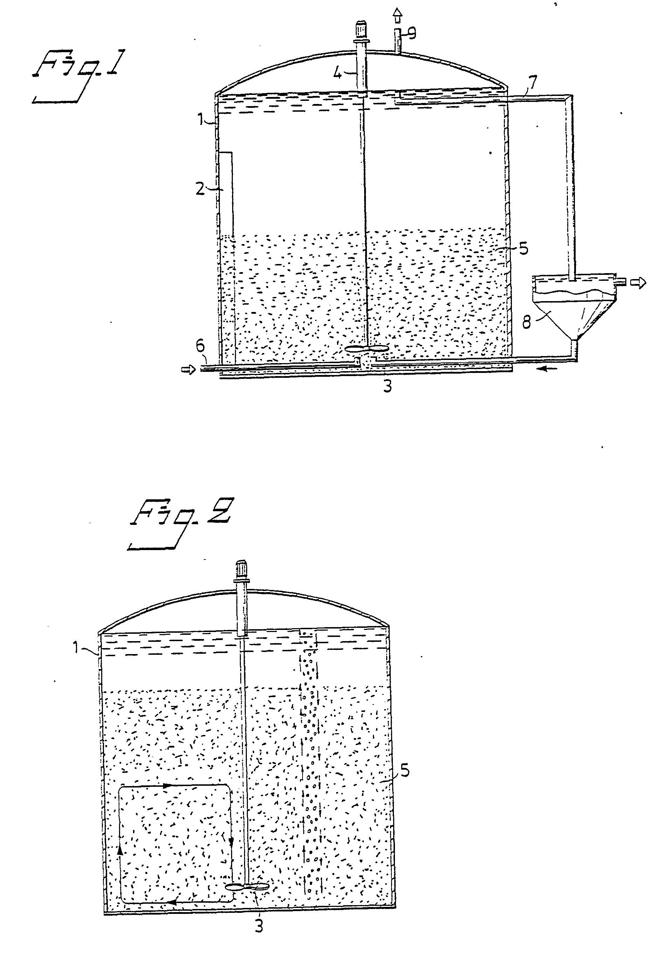

The embodiment of the apparatus according to this invention shown in Fig. 1 of the drawings includes a circular-cylindrical reaction vessel 1 having a planar bottom and a curved top, the vessel being provided with interior vertical baffles 2 which may be present in a number of 1—10. It is preferred to use a plurality of baffles evenly distributed around the inner wall of the vessel. The reaction vessel is furthermore provided with a central stirrer 3 driven by an externally placed motor through a gas-tight throughlet 4 at the upper end of vessel 1.

Reaction vessel 1 contains a movable bed 5 of an inorganic granular material which at rest in the embodiment shown occupies about half of the volume of the reaction vessel. The reaction vessel furthermore contains water as a continuous phase up to the level shown in Fig. 1.

The supply of substrate, i.e. material to be treated, takes place though an inlet conduit 6 opening directly under the stirrer 3. Treated liquid is removed from the reaction vessel through an outlet conduit 7 and is transferred to a separation device 8 whereby separation bio sludge is separated and returned to the reaction vessel to a place immediately below stirrer 3.

The function of the apparatus described above is briefly as follows.

On the suspended bed material of inorganic material which may be sand, calcium carbonate, calcium hydroxide or dolomite having a grain size within the range 0.05 to 5 mm, present in the reaction vessel, a flora of anaerobic microorganisms will grow in a thin layer. Thereby, the microorganisms will convert supplied organic compounds to different types of products in dependence on the type of substrate and the type of microorganisms.

For the purpose of transferring the substrate to the microorganisms bound to the inorganic grains of the bed material the substrate is transferred through inlet conduit 6 to a place immediately below the stirrer 3 which is centrally positioned in the bed (Fig. 1).

When under operation stirrer 3 is brought into rotation a flow of liquid and bed grains will be pumped around in the reaction vessel, the substrate coming into intimate contact with the remaining contents of the reaction vessel. The flow created by stirrer 3 is horizontal and downwardly directed, in view of which a strong flow will be obtained along the bottom of the reaction vessel and upwardly along the walls of the reaction vessel. This flow means that the movable bed will extend, the expansion being further increased by the generation of a gas which takes place when the substrate comes into contact with the microorganisms supported by the bed. In this manner a very even and homogeneous distribution of the substrate in the bed will be obtained whereby effective utilisation of the microorganisms will take place.

In Fig. 2 the flows provided by the mechanical mixing by stirrer 3 and by the gases generated in the microbiological reaction are illustrated. In view of the direction of the flow created by the stirrer there is obtained efficient distribution of the substrate horizontally at the bottom of the reaction vessel. In view of the gas generation and the entrainment of liquid phase with the gas bubbles there is obtained a good mixing in vertical direction, as is clear from Fig. 2.

The gas generation in the apparatus described is of the order of 1—100 $m^3$ of gas per $m^3$ reaction vessel per day, and the generated gases vary in dependence of type of substrate and microorganism, and may be carbon dioxide, methane gas or a mixture thereof, or nitrogen. By modifying the height of the baffles and the rotation of the mechanical stirrer the movable bed can be brought to expand to the desired level. By discharging a liquid phase from the surface at the upper end of the reaction vessel where the movable bed does not reach by the expansion the microorganisms may to a great extent be maintained in the system. The quantity of microorganisms maintained bound to the bed material may vary from 1—50 g of DS/l volume of reaction vessel.

Part of the microorganisms are found in the form of microflocks in the liquid phase. This part will not be separated in the reaction vessel but is carried along with the discharged liquid flow. By means of separation device 8 they can be separated from the liquid and returned to the reaction vessel. in this way the microorganisms may be maintained suspended in a quantity corresponding to 5—10 g TS/l (total suspended solids). In toto one can obtain a total content of microorganisms bound to the movable bed and suspended of about 60 g DS/l, which is 6—12 times of what can be obtained with conventional technique.

The invention is not delimited to the embodiment described above. Thus, the ratio between height and diameter of the reaction vessel may vary from 5:1 to 1:5. Moreover, the details of the construction described can be varied and modified in a manner obvious to those skilled in the art.

## Claims

1. A process for converting organic material in a microbiological way under gas generation, characterized thereby that the conversion is carried out in a reaction vessel by means of microorganisms which are grown on a movable bed of inorganic granular material and by means of microorganisms freely in micro flocks in a continuous aqueous phase, that the substrate for the microorganisms is supplied to the movable bed stirred by a mechanical stirrer and that the gases generated by the conversion are utilized for expanding the movable bed, the free micro flocks being externally separated from discharged liquid and returned to the reaction vessel.

2. A process according to claim 1, characterized thereby that the inorganic granular material has a grain size within the range of about 0.05—5 mm.

3. A process according to claim 1 or 2, characterized thereby that the granular inorganic material is constituted by sand or calcium carbonate.

4. An apparatus for converting organic material in liquid phase in a microbiological way, including a reaction vessel (1), a stirrer (3) arranged therein, means (6) for supply of liquid containing substrate, means (7, 8) for discharge of liquid and for recirculation of free active material, and a movable bed (5) of inorganic granular material positioned in the reaction vessel and intended for carrying growing microorganisms, characterized thereby that said means (6) for supply of liquid containing substrate is arranged in direct connection with the stirrrer (3).

5. Apparatus according to claim 4, characterized thereby that the inorganic granular material has a grain size within the range of about 0.05—5 mm.

6. Apparatus according to claim 4 or 5, characterized thereby that the granular inorganic material is constituted by sand or calcium carbonate.

7. Apparatus according to any of claims 4—6, characterized thereby that the stirrer consists of a radial or axial turbine (3) placed at the lower end of the reaction vessel and generating a downward flow.

8. Apparatus according to any of claims 4—7, characterized thereby that the movable bed (5) at rest occupies 20—80% of the volume of the reaction vessel (1).

9. An apparatus according to any of claims 4—8, characterized thereby that the reaction vessel (1) is provided with interior vertical baffles (2) extending over 20—100% of the height of the vessel.

## Revendications

1. Procédé de conversion d'une matière organique par voie microbiologique avec formation de gaz, caractérisé en ce que la conversion est effectuée dans un réacteur au moyen de micro-organismes qui sont cultivés sur un lit mobile de matériaux granulaires inorganiques et au moyen de micro-organismes que se trouvent à l'état libre sous forme de micro-flocons dans une phase aqueuse continue; que le substrat pour les micro-organismes est apporté au lit mobile agité par un agitateur mécanique et que les gaz formés par la conversion sont utilisés pour l'expansion du lit mobile, les micro-flocons libres étant séparés à l'extérieur, du liquide évacué et renvoyés au réacteur.

2. Procédé selon la revendication 1, caractérisé en ce que le matériau granulaire inorganique a une dimension de grains comprise entre environ 0,05 et 5 mm.

3. Procédé selon la revendication 1 ou 2, caractérisé en ce que le matériau inorganique granulaire est constitué par du sable ou du carbonate de calcium.

4. Appareil pour la conversion d'une matière organique en phase liquide par voie microbiologique, comprenant un réacteur (1), un agitateur (3) disposé à l'intérieur de celui-ci, des moyens (6) pour apporter le liquide contenant le substrat, des moyens (7, 8) pour évacuer le lquide et pour recycler la matière active libre et un lit mobile (5) d'un matériau granulaire inorganique placé dans le réacteur et destiné à servir de support pour la culture de micro-organismes, caractérisé en ce que ces moyens (6) pour apporter le liquide contenant le substrat sont disposés en relation directe avec l'agitateur (3).

5. Appareil selon la revendication 4, caractérisé en ce que le matériau granulaire inorganique a une dimension de grains comprise entre environ 0,05 et 5 mm.

6. Appareil selon la revendication 4 ou 5, caractérisé en ce que le matériau inorganique granulaire est constitué par du sable ou du carbonate de calcium.

7. Appareil selon l'une quelconque des revendications 4 à 6, caractérisé en ce que l'agitateur consiste en une turbine radiale ou axiale (3) placée à l'extrémité inférieure du réacteur et engendrant un courant descendant.

8. Appareil selon l'une quelconque des revendications 4 à 7, caractérisé en ce que le lit mobile occupe, au repos, 20 à 80% du volume du réacteur (1).

9. Appareil selon l'une quelconque des revendications 4 à 8 caractérisé en ce que le réacteur (1) est muni de chicanes verticales intérieures (2) qui s'étendent sur 20 à 100% de la hauteur du réacteur.

**Patentansprüche**

1. Verfahren zur Umwandlung von organischem Material auf mikrobiologischem Weg unter Gaserzeugung, dadurch gekennzeichnet, daß die Umwandlung in einem Reaktionsbehälter mit Hilfe von Mikroorganismen, die auf einer beweglichen Schicht von anorganischem körnigem Material gezüchtet werden, und mit Hilfe als Mikroflocken frei in zusammenhängender wäßriger Phase befindlicher Mikroorganismen durchgeführt wird, daß das Substrat für die Mikroorganismismen der mit einem mechanischen Rührer gerührten beweglichen Schicht zugeführt wird und daß die durch die Umwandlung erzeugten Gase für die Ausdehnung der beweglichen Schicht benutzt werden, wobei die freien Mikroflocken außerhalb von der entnommenen Flüssigkeit abgetrennt und zu dem Reaktionsbehälter zurückgeführt werden.

2. Verfahren nach Anspruch 1, dadurch gekennzeichnet, daß das anorganische körnige Material eine Korngröße im Bereich von etwa 0,05 bis 5 mm hat.

3. Verfahren nach Anspruch 1 oder 2, dadurch gekennzeichnet, daß das körnige anorganische Material aus Sand oder Calciumcarbonat besteht.

4. Vorrichtung zur Umwandlung von organischem Material in flüssiger phase auf mikrobiologischem Weg mit einem Reaktionsbehälter (1), einem darin angeordneten Rührer (3), Einrichtungen (6) zur Zuführung von substrathaltiger Flüssigkeit, Einrichtungen (7, 8) zur Entnahme von Flüssigkeit und zur Rückführung von freiem aktivem Material und einer beweglichen Schicht (5) von anorganischem körnigem Material, das in dem Reaktionsbehälter angeordnet ist und zum Tragen darauf wachsender Mikroorganismen bestimmt ist, dadurch gekennzeichnet, daß die Einrichtung (6) zur Zuführung von substrathaltiger Flüssigkeit in direkter Verbindung mit dem Rührer (3) angeordnet ist.

5. Vorrichtung nach Anspruch 4, dadurch gekennzeichnet, daß das anorganische körnige Material eine Korngröße im Bereich von etwa 0,05 bis 5 mm hat.

6. Vorrichtung nach Anspruch 4 oder 5, dadurch gekennzeichnet, daß das körnige anorganische Material aus Sand oder Calciumcarbonat besteht.

7. Vorrichtung nach einem der Ansprüche 4 bis 6, dadurch gekennzeichnet, daß der Rührer aus einer radialen oder axialen Turbine (3) besteht, die am unteren Ende des Reaktionsbehälters angeordnet ist und einen Abwärtsstrom erzeugt.

8. Vorrichtung nach einem der Ansprüche 4 bis 7, dadurch gekennzeichnet, daß die bewegliche Schicht (5) im Ruhezustand 20 bis 80% des Volumens des Reaktionsbehälters (1) einnimmt.

9. Vorrichtung nach einem der Ansprüche 4 bis 8, dadurch gekennzeichnet, daß der Reaktionsbehälter (1) mit inneren senkrechten Prallplatten (2) versehen ist, die sich über 20 bis 100% der Höhe des Behälters erstrecken.

Fig.1

Fig.2